# EUROPÄISCHE PATENTANMELDUNG

(11) **EP 3 424 914 A1**
(43) Veröffentlichungstag der Anmeldung: **09.01.2019**
(21) Anmeldenummer: 17179747.5
(22) Anmeldetag: 05.07.2017
(51) Int. Cl.: C07D 307/46

(54) **VERFAHREN ZUR EXTRAKTION VON 5-HYDROXYMETHYLFURFURAL (5-HMF)**

(71) Anmelder: AVALON Industries AG, 6304 Zug (CH)
(72) Erfinder: MORTATO, Mariangela, 4051 Basel Stadt (CH); BADOUX, Francois, 6343 Rotkreuz (CH); DESROCHES, Marie, 98002 Zürich (CH); MORLEY, Philip, 6330 Cham (CH)
(74) Vertreter: Geitz Truckenmüller Lucht Christ

(57) **Zusammenfassung**

Gegenstand der Erfindung ist ein Verfahren zur Gewinnung von 5-HMF aus einer wässrigen Lösung, das die Schritte
(A) Bereitstellen der wässrigen Lösung, enthaltend 5-HMF, und eines organischen Extraktionsmittels,
(B) Flüssig-flüssig-Extraktion, bei der die wässrige Lösung mit dem organischen Extraktionsmittel unter Ausbildung einer Dispersion durchmischt wird, wobei das organische Extraktionsmittel so bemessen ist, dass sich beim Dispergieren eine wässrige Raffinatphase b1 als disperse Phase und eine organische Phase b2 als kontinuierliche Phase ausbildet, wobei eine im Wesentlichen von 5-HMF freie wässrige Raffinatphase b1 und eine mit 5-HMF beladene organische Phase b2 erhalten werden,
(C) Abtrennen der organischen Phase b2 von der wässrigen Raffinatphase b1,
umfasst.

## Beschreibung

Die Erfindung betrifft ein Verfahren zur Gewinnung von 5-Hydroxymethylfurfural (5-HMF) aus wässrigen Lösungen.

Das substituierte Furan 5-Hydroxymethylfurfural (HMF) 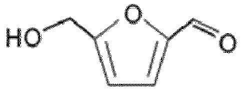 stellt eine wichtige Plattformchemikalie dar und hat als Ausgangsverbindung für zahlreiche Synthesen erhebliche Bedeutung erlangt. 5-HMF lässt sich durch die Behandlung von cellulosehaltiger Biomasse oder von Hexosen unter hydrothermalen Bedingungen erhalten. Die Herstellung von 5-HMF ist bekannt und sieht vor, das Ausgangsmaterial in wässrigem Medium Druck und erhöhter Temperatur auszusetzen. Die damit einhergehende Zersetzung der Hexosen zeichnet sich durch Dehydratisierung und Decarboxylierung sowie Cyclisierung aus. Bei geeigneter Prozessführung handelt es sich bei dem von einer Hydrothermalen Karbonisierung abgeleiteten Verfahren um einen wertvollen Lieferanten für 5-HMF. Die Vielzahl an hierbei sequenziell und parallel ablaufenden chemischen Reaktionen führt zu einer Reihe an Zwischen- und Nebenprodukten, sodass 5-HMF neben weiteren, die Reinheit und Ausbeute vermindernden Verbindungen in einer auch als Prozesswasser bezeichneten wässrigen Lösung vorliegt.

Aufgrund seiner Struktur zeigt 5-HMF eine praktisch unbegrenzte Löslichkeit in Wasser oder in wässrigen Lösungen. Zudem liegt in einer aus dem Herstellungsprozess von 5-HMF stammenden wässrigen Lösung eine Vielzahl weiterer, ebenfalls sehr gut wasserlöslicher Verbindungen vor.

Vor diesem Hintergrund liegt der vorliegenden Erfindung die Aufgabe zugrunde, ein effizientes Verfahren zur Gewinnung von 5-HMF aus wässrigen Lösungen bereitzustellen.

Dies gelingt durch ein Verfahren gemäß den Merkmalen des Anspruchs 1. Weitere sinnvolle Ausgestaltungen des Verfahrens können den Unteransprüchen entnommen werden.

Das Verfahren zur Gewinnung von 5-HMF aus einer wässrigen Lösung umfasst die Schritte
(A) Bereitstellen der wässrigen Lösung, enthaltend 5-HMF, und eines organischen Extraktionsmittels,
(B) Flüssig-flüssig-Extraktion, bei der die wässrige Lösung mit dem organischen Extraktionsmittel unter Ausbildung einer Dispersion durchmischt wird, wobei das organische Extraktionsmittel so bemessen ist, dass sich beim Dispergieren eine wässrige Raffinatphase b1 als disperse Phase und eine organische Phase b2 als kontinuierliche Phase ausbildet, wobei eine im Wesentlichen von 5-HMF freie wässrige Raffinatphase b1 und eine mit 5-HMF beladene organische Phase b2 erhalten werden,
(C) Abtrennen der organischen Phase b2 von der wässrigen Raffinatphase b1.

Überraschenderweise kann durch die Zurverfügungstellung des erfindungsgemäßen Verfahrens aus einer wässrigen Lösung 5-HMF mit hoher Ausbeute und hoher Reinheit extrahiert werden.

Im Allgemeinen werden mit dem erfindungsgemäßen Verfahren wässrige Lösungen aufgearbeitet, die neben 5-HMF herstellungsbedingt unter anderem Fructose, Glucose, Zuckerintermediate, Formaldehyd und Carbonsäuren wie Lävulinsäure, Essigsäure und Ameisensäure enthalten. Es wurde gefunden, dass durch die erfindungsgemäße Durchführung des Verfahrens eine effiziente Abtrennung des 5-HMF von diesen Verbindungen ermöglicht wird.

Unter einer "Flüssig-flüssig-Extraktion" wird erfindungsgemäß eine Extraktion eines in einem flüssigen Lösungsmittel gelösten Stoffes mittels eines zweiten flüssigen Lösungsmittels verstanden.

Unter dem Begriff "wässrige Lösung" wird eine Lösung verstanden, die überwiegend Wasser als Lösungsmittel enthält. Neben dem in der Lösung gelösten 5-HMF können weitere Substanzen in der Lösung gelöst oder mit dieser vermischt vorliegen. Die wässrige Lösung kann zudem Substanzen enthalten, die die Extraktion oder die Stabilität des 5-HMF verbessern oder zu bevorzugten Eigenschaften der wässrigen Lösung, wie beispielsweis pH-Wert führen.

Bevorzugt werden in dem erfindungsgemäßen Verfahren wässrige Lösungen aufgearbeitet, welche zwischen 15 und 20 Gew.% 5-HMF aufweisen. Die wässrige Lösung kann jedoch auch geringere oder höhere Mengen an 5-HMF enthalten.

Im Allgemeinen liegt das Volumenverhältnis der mit 5-HMF beladenen organischen Phase b2 zu der wässrigen Raffinatphase b1 über 1 l pro I, damit sich die organische Phase b2 als kontinuierlich ausbildet. Die Auswahl geeigneter Volumenverhältnisse liegt im Können eines Fachmanns.

Zur Durchführung des erfindungsgemäßen Verfahrens geeignete Extraktionsmittel weisen eine ausreichend große Mischungslücke mit der wässrigen Lösung auf und einen für 5-HMF hinreichend hohen Verteilungskoeffizienten. Geeignete Extraktionsmittel sind organische Extraktionsmittel, die beispielhaft bereits in dem Fachartikel von Blumenthal et al. in ACS Sustainable Chem. Eng. 2016, 4, 228-235 beschrieben sind. Durch eine innige Vermischung von wässriger Lösung und organischem Extraktionsmittel wird hierbei der Massetransfer zwischen wässriger Lösung und organischem Extraktionsmittel gefördert.

Vorteilhafterweise kann mit dem erfindungsgemäßen Verfahren insbesondere aus einer wässrigen Lösung, die sowohl 5-HMF als auch Fructose, Glucose, Zuckerintermediate und Carbonsäuren wie Lävulinsäure, Essigsäure und Ameisensäure enthält, 5-HMF schonend abgetrennt werden.

Vorzugsweise wird in Schritt (B) eine wässrige Raffinatphase b1 enthaltend Fructose, Glucose, Zuckerintermediate und Carbonsäuren erhalten.

Bevorzugt wird in Schritt (C) eine mit 5-HMF beladene organische Phase b2 abgetrennt, in der 5-HMF mit einer Reinheit von 86% bis 90%, besonders bevorzugt mit einer Reinheit von 88% bis 90% vorliegt.

Nach einer vorteilhaften Ausgestaltung des Verfahrens ist das organische Extraktionsmittel ausgewählt aus
- den halogenierten Kohlenwasserstoffen und insbesondere dem Dichlormethan und dem Chloroform,
- den Ketonen und insbesondere dem Methylisobutylketon,
- den aliphatischen Kohlenwasserstoffen und insbesondere dem 1-Butanol und dem 2-Butanol,
- den Furan-Derivaten und insbesondere dem 2-Methyltetrahydrofuran und dem Tetrahydrofuran,
- den alkylierten Phenolen, insbesondere dem o-Propylphenol und dem o-Isopropylphenol,
oder aus Mischungen von diesen.

Das organische Extraktionsmittel wird unter Beachtung der folgenden Voraussetzungen ausgewählt:
- es darf unter den gewählten Bedingungen so wenig wie möglich mit der wässrigen Lösung mischbar sein,
- es muss einen hohen Verteilungskoeffizienten für 5-HMF aufweisen und damit ein gutes Lösungsmittel für 5-HMF sein, und
- es weist eine ausreichend hohe Differenz in seiner Dichte zu der wässrigen Lösung auf, sodass eine schnelle und effiziente Phasentrennung gefördert wird.

Mischungen aus den organischen Lösungsmitteln sind ebenfalls gut geeignet, solange das Gemisch die hierin für das Extraktionsmittel beschriebenen Eigenschaften aufweist, insbesondere zur Extraktion von 5-HMF dienen kann.

Ein besonders bevorzugtes Extraktionsmittel ist Dichlormethan. Nach einer weiteren vorteilhaften Ausgestaltung der Erfindung ist das organische Extraktionsmittel ausgewählt aus Dichlormethan und Mischungen enthaltend Dichlormethan. Die Verwendung von Dichlormethan als Extraktionsmittel bringt große Vorteile mit sich. So wird nach dem erfindungsgemäßen Verfahren 5-HMF in guter Ausbeute und hoher Reinheit gewonnen. Zusätzlich zu diesem vorteilhaften Ergebnis macht sich das erfindungsgemäße Verfahren die folgenden positiven Eigenschaften des Dichlormethans zu eigen: Es wurde gefunden, dass das Dichlormethan eine geringe Tendenz dazu aufweist, Abbau-Reaktionen von 5-HMF wie beispielsweise eine Polymerisation und damit eine Oligomerbildung zu katalysieren. Hierdurch ergibt sich 5-HMF mit einem besonders niedrigen Oligomergehalt. Zudem besitzt höhere Dichte, sodass sich zwei Phasen ausbilden, welche leicht trennbar sind. Dichlormethan ist zudem nicht-entflammbar und minimiert lösungsmittelbedingte Risiken für Umwelt und Gesundheit. Von weiterem Vorteil ist ein relativ niedriger Siedepunkt (40°C), der neben einer energiesparenden, einfachen thermischen Entfernung von Dichlormethan zu einer Verminderung eines thermisch bedingten Abbaus von 5-HMF, insbesondere zu Oligomeren, führt.

Es war nicht zu erwarten, dass in Dichlormethan ein derart optimales Extraktionsmittel zur Durchführung des erfindungsgemäßen Verfahrens gefunden werden könnte, da es insbesondere in Blumenthal et al. und den mit dem Fachartikel in Verbindung veröffentlichten Zusatzinformationen, die eine Vielzahl an geeigneten Lösungsmitteln vorschlagen, überhaupt nicht erwähnt wird. Ebenso verhält es sich mit Chloroform, das aufgrund seiner Eigenschaften ebenfalls bevorzugt ist.

Auch Gemische, enthaltend Dichlormethan und/oder Chloroform mit anderen organischen Lösungsmitteln können nützlich sein und sind daher ebenfalls bevorzugt.

Weitere substituierte Furane, wie Furyl-Hydroxymethyl-Keton (FHK) und Furfural, sowie lösliche Furan-Oligomere, welche neben 5-HMF, Fructose, Glucose, Zuckerintermediaten, Formaldehyd und Carbonsäuren in der wässrigen Lösung enthalten sein können, weisen aufgrund ihrer Struktur sehr ähnliche chemische und physikalische Eigenschaften auf wie 5-HMF. Es wurde gefunden, dass sich diese Verbindungen ebenfalls in der organischen Phase b2 anreichern können. Es wurde weiterhin gefunden, dass diese Verbindungen von 5-HMF abgetrennt werden können und die Reinheit des 5-HMF nochmals erhöht werden kann, indem vor der Durchführung des Schrittes (B) die 5-HMF, FHK, Furfural sowie lösliche Furan-Oligomere enthaltende wässrige Lösung einer Flüssig-flüssig-Extraktion mit umgekehrter Dispergierrichtung unterzogen wird.

Nach einer weiteren vorteilhaften Ausgestaltung des Verfahrens wird vor der Durchführung von Schritt (B) eine Flüssig-flüssig-Extraktion mit umgekehrter Dispergierrichtung durchgeführt, bei der die wässrige Lösung mit dem organischen Extraktionsmittel unter Ausbildung einer Dispersion durchmischt wird, wobei das organische Extraktionsmittel so bemessen ist, dass sich beim Dispergieren eine wässrige Raffinatphase als kontinuierliche Phase i1 und eine organische Phase i2 als disperse Phase ausbildet.

"Flüssig-flüssig-Extraktion mit umgekehrter Dispergierrichtung" bedeutet hierin, dass sich bei der Flüssig-flüssig-Extraktion, die dem Schritt (B) des Verfahrens vorangestellt wird, die Phase als kontinuierlich ausbildet, die im Schritt (B) dispers ausgebildet ist und umgekehrt.

Bei der Flüssig-flüssig-Extraktion mit umgekehrter Dispergierrichtung, die im Folgenden auch als inverse Extraktion bezeichnet wird, werden eine im Wesentlichen von FHK, Furfural sowie löslichen Furan-Oligomeren freie wässrige Raffinatphase i1 und eine mit FHK, Furfural sowie löslichen Furan-Oligomeren beladene organische Phase i2 erhalten. Die wässrige Raffinatphase i1 enthält 5-HMF und kann als Raffinat aus der inversen Extraktion ab- und der Flüssig-flüssig-Extraktion in Schritt (B) des erfindungsgemäßen Verfahrens zugeführt werden.

Bisher wurde nicht gezeigt, dass sich FHK, Furfural und lösliche Furan-Oligomere, die eine derartig hohe strukturelle Ähnlichkeit zu 5-HMF aufweisen, von diesem abtrennen lassen.

Das Volumenverhältnis der das 5-HMF enthaltenden wässrigen Raffinatphase i1 zu der organischen Phase i2 liegt im Allgemeinen über 1 l pro I, damit sich die wässrige Raffinatphase i1 als kontinuierlich ausbildet.

Die wässrige Raffinatphase i1 enthält vorzugsweise den wesentlichen Anteil an 5-HMF aus der wässrigen Lösung. Sofern bei der inversen Extraktion ein gewisser Anteil des 5-HMF ebenfalls mit FHK, Furfural und den löslichen Furan-Oligomeren in die organische Phase i2 überführt wird, resultiert dies in einer geringeren Ausbeute an 5-HMF in der wässrigen Raffinatphase i1. Es wurde gefunden, dass das Problem behoben werden kann, indem, bevorzugt bei der Durchführung der inversen Extraktion, 5-HMF aus der organischen Phase i2 rückextrahiert wird.

Nach einer weiteren vorteilhaften Ausgestaltung der Erfindung wird das 5-HMF vollständig oder partiell aus der organischen Phase i2 mit einem polaren Lösungsmittel in die wässrige Raffinatphase i1 rückextrahiert.

Polare Lösungsmittel sind erfindungsgemäß wässrige Lösungsmittel, inklusive Wasser, oder polare aprotische oder protische organische Lösungsmittel sowie Mischungen von diesen, in denen 5-HMF eine möglichst gute Löslichkeit aufweist.

Unter "vollständiges oder partielles Rückextrahieren" wird erfindungsgemäß verstanden, dass 5-HMF im Wesentlichen, vorzugsweise mindestens zu 40 Gew.% bis 100 Gew.% in eine wässrige Phase rückgeführt wird. Erfindungsgemäß ist die wässrige Phase die wässrige Raffinatphase i1 der inversen Extraktion.

Um eine effiziente Rückextraktion zu fördern, ist die Konzentration des 5-HMF vor der Rückextraktion im polaren Lösungsmittel geringer als in der organischen Phase i2, bevorzugt ist das polare Lösungsmittel frei von 5-HMF. Vorzugsweise ist das polare Lösungsmittel Wasser, eine wässrige Lösung oder das polare Lösungsmittel ist zumindest vollständig mit Wasser mischbar.

Nach einer weiteren vorteilhaften Ausführung des Verfahrens wird das 5-HMF rückextrahiert, indem die organische Phase i2 mit dem polaren Lösungsmittel in innigen Kontakt gebracht wird, wobei 5-HMF aus der organischen Phase i2 in das polare Lösungsmittel überführt wird, und das mit 5-HMF beladene polare Lösungsmittel mit der wässrigen Raffinatphase i1 vereint wird.

Bevorzugt wird bei der Durchführung des erfindungsgemäßen Verfahrens, umfassend eine inverse Extraktion und eine Rückextraktion, in Schritt (C) eine mit 5-HMF beladene organische Phase b2 abgetrennt, in der 5-HMF mit einer Reinheit von 91% bis 99%, bevorzugt mit einer Reinheit von 97% und mehr, vorzugsweise von 97% bis 99%, vorliegt.

Nach einer vorteilhaften Ausgestaltung der Erfindung werden eine oder mehrere von dem erfindungsgemäßen Verfahren umfasste Extraktionen im Gegenstrom vorgenommen. Jeder Extraktionsschritt kann prinzipiell kontinuierlich oder satzweise durchgeführt werden. Geeignet für eine satzweise Extraktion sind beispielsweise Rührkessel. Bevorzugt wird jedoch die Extraktion, respektive die Flüssig-flüssig-Extraktion in Schritt (B), die inverse Extraktion mit oder ohne Rückextraktion und/oder eine separate Rückextraktion, kontinuierlich im Gegenstrom durchgeführt, da dies zu einem beschleunigten Massetransfer zwischen wässriger Lösung und organischem Extraktionsmittel führt. Es sind auch Kombinationen aus satzweiser und kontinuierlicher Extraktion von dem erfindungsgemäßen Verfahren umfasst.

Bei der Gegenstromextraktion werden wässrige Lösung und organisches Extraktionsmittel einer Flüssig-flüssig-Gegenstrom-Extraktorkolonne an entgegengesetzten Enden zugeführt und beide durchströmen diese kontinuierlich im Gegenstrom. Idealerweise läuft der Trennprozess stufenweise ab, wobei die Raffinatphase (abgereicherte wässrige Lösung) einer Stufe zum Zulauf der nächsten Stufe wird und die Extraktphase (angereichertes Extraktionsmittel) einer Stufe zum Extraktionsmittel der vorigen Stufe wird. Hierdurch kann eine hohe Anreicherung von zu extrahierender Substanz im Extraktionsmittel gefördert werden. Es sind verschiedene Bauformen von Flüssig-flüssig-Gegenstrom-Extraktorkolonnen zur Durchführung des Verfahrens bekannt. In Frage kommen beispielsweise Sprühkolonnen, Füllkörperkolonnen, Siebbodenkolonnen, pulsierte Füll- oder Siebbodenkolonnen, Drehscheibenkolonnen sowie Mixer-Settler-Batterie-Kolonnen. In einer kontinuierlichen Flüssig-flüssig-Gegenstrom-Extraktorkolonne kann ein organisches Extraktionsmittel mit einer höheren Dichte als die wässrige Lösung in einer vertikalen Kolonne an einer Stelle oberhalb der Zuleitungsstelle, an der die wässrige Lösung eingebracht wird, zugeleitet werden. Bei einem organischen Extraktionsmittel mit einer geringeren Dichte erfolgt die Zuführung unterhalb der Einleitungsstelle für die wässrige Lösung. Je nach Verfahrensführung kann eine Extraktoranlage betrieben werden mit einer einzigen Flüssig-flüssig-Gegenstrom-Extraktorkolonne oder mehrere gleiche oder unterschiedliche Flüssig-flüssig-Gegenstrom-Extraktorkolonnen können in einer Extraktoranlage kombiniert werden.

Nach einer weiteren vorteilhaften Ausgestaltung des Verfahrens werden die Schritte (A) bis (C) bei Raumtemperatur durchgeführt. Gegebenenfalls kann die inverse Extraktion ebenfalls bei Raumtemperatur durchgeführt werden. Hierdurch kann ein besonders schonendes Verfahren zur Gewinnung von 5-HMF zur Verfügung gestellt werden. Die Schritte können auch bei anderen Temperaturen betrieben werden, wobei die Grenzen nach unten durch die Viskositätszunahme in den Phasen und nach oben durch den Siedepunkt des organischen Extraktionsmittels und den mit steigender Temperatur zunehmenden Abbau des 5-HMF zu Oligomeren gegeben sind.

Nach einer weiteren vorteilhaften Ausgestaltung der Erfindung umfasst das erfindungsgemäße Verfahren den folgenden weiteren Schritt
(D) partielles oder vollständiges Entfernen des in der organischen Phase b2 enthaltenen organischen Extraktionsmittels.

Nach der Abtrennung der organischen Phase b2 in Schritt (C) ist es für vielfältige Anwendungen vorteilhaft, die mit 5-HMF beladene organische Phase b2 aufzukonzentrieren oder das 5-HMF von dem Extraktionsmittel zu isolieren. Eine vollständige Entfernung des Extraktionsmittels, beispielsweise durch Trocknung oder Kristallisation, sowie eine partielle Entfernung sind dem Fachmann bekannt. Vorzugsweise wird das in der organischen Phase b2 enthaltene Extraktionsmittel partiell oder vollständig durch Abdampfen entfernt. Bei dem partiellen Entfernen kann das Abdampfen bis zu einer gewünschten Konzentration an 5-HMF im Extraktionsmittel geführt werden. Insbesondere vorteilhaft hierbei ist, dass sich eine optimal konzentrierte Extraktionsmittel-5-HMF-Lösung einstellen lässt, die eine besonders geringe Tendenz von 5-HMF zur Oligomerbildung zeigt. Besonders bevorzugt wird das Abdampfen so geführt, dass die verbleibende Lösung ca. 15 Gew.% bis 40 Gew.% 5-HMF, bevorzugt 15 Gew.% bis 20 Gew.% 5-HMF aufweist.

Bevorzugt wird das Extraktionsmittel beim Abdampfen zumindest teilweise mit einem Verdampfer entfernt, welcher eine kurze Verweilzeit des mit 5-HMF beladenen Extraktionsmittels in der Eindampfungsstufe gestattet. Hierzu geeignet sind Kurzweg-Verdampfer und Fallfilm-Verdampfer. Bevorzugt sind Dünnschicht-Verdampfer. Die Verdampfer können auch in Kombination miteinander und/oder mit weiteren Einrichtungen zur Trennung von 5-HMF und Extraktionsmittel, wie beispielsweise Rektifikationskolonnen, eingesetzt werden.

Besonders bevorzugt erfolgt das Abdampfen des Extraktionsmittels bei unter 40°C, insbesondere bevorzugt erfolgt das Abdampfen bei 10°C bis 30°C und bei reduziertem Druck. Vorteilhafter ist eine Temperatur von 15°C bis 25°C und ein reduzierter Druck.

Nach einer weiteren vorteilhaften Ausgestaltung der Erfindung wird eingangs des erfindungsgemäßen Verfahrens eine Filtration der wässrigen Lösung vorgenommen. Beispielsweise können je nach Bedingungen der hydrothermalen Behandlung die in der wässrigen Lösung enthaltenen Verbindungen mehr oder weniger stark polymerisiert vorliegen. Zur Entfernung von in der wässrigen Lösung vorhandenen Partikeln wie Oligomere kann es dabei insbesondere sinnvoll sein, die wässrige Lösung eingangs des Verfahrens einer separaten Fest-Flüssig-Filtration zuzuführen. Hierzu wird beispielsweise eine Oberflächen-, Tiefenfiltration und/oder eine Membranfiltration durchgeführt. Auch kann eine Filtration dazu genutzt werden, mehr oder weniger große Anteile an löslichen Furan-Oligomeren aus der wässrigen Lösung, die mit dem erfindungsgemäßen Verfahren aufgearbeitet wird, zu entfernen.

Die vorstehend beschriebene Erfindung wird im Folgenden unter Bezugnahme auf die beigefügten Figuren näher erläutert.

Es zeigen:
Fig. 1 eine schematische Darstellung einer zur Durchführung des erfindungsgemäßen Verfahrens geeigneten Apparatur und des Ablaufs einer Flüssig-Flüssig-Extraktion im Gegenstrom mit einer Extraktionskolonne.
Fig. 2 eine schematische Darstellung einer weiteren zur Durchführung des erfindungsgemäßen Verfahrens geeigneten Apparatur und des Ablaufs von inverser Extraktion gefolgt von einer Flüssig-flüssig-Extraktion im Gegenstrom mit zwei Extraktionskolonnen.
Fig. 3 eine schematische Darstellung eines Verdampfers und der Aufkonzentrierung von mit 5-HMF beladenem Dichlormethan.

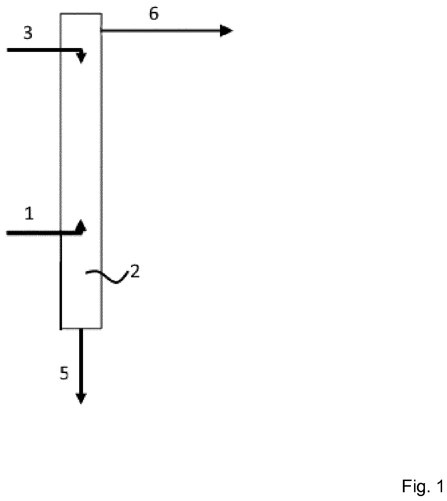

In Fig. 1 ist schematisch der Ablauf einer Extraktion von 5-HMF aus einer verdünnten wässrigen Lösung, welche auf an sich bekannte Weise durch hydrothermale Behandlung von cellulosehaltiger Biomasse gewonnen wurde, dargestellt. Die Anteile der in der wässrigen Lösung enthaltenen Komponenten wie Furyl-Hydroxymethyl-Keton (FHK mit der IUPAC Bezeichnung 1-(2-Furyl)-2-hydroxyethanon), Furfural und lösliche Furan-Oligomere im Verhältnis zu 5-HMF können der Tabelle 1 (HMF-Lösung) entnommen werden. Die wässrige Lösung wird nach einer Filtration über die Zuleitung (1) im Bodenbereich einer Extraktionskolonne (2) eingespeist. Das Extraktionsmittel Dichlormethan wird über die Zuleitung (3) am Kopf der Extraktionskolonne (2) eingeführt und bei Raumtemperatur im Gegenstrom zur wässrigen Lösung geleitet.

Das mit 5-HMF angereicherte Dichlormethan, welches über eine Ableitung (5) am Boden der Extraktionskolonne (2) als Extrakt entnommen wird, enthält ca. 7,5 Gew.% bis 10 Gew.% 5-HMF mit einer Reinheit von 88% bis 90%. Weitere, in geringeren Mengen im Extrakt enthaltene substituierte Furane sind Furyl-Hydroxymethyl-Keton (FHK), Furfural und lösliche Furan-Oligomere. Die Anteile der im Extrakt enthaltenen Komponenten im Verhältnis zu 5-HMF können der Tabelle 1 (DCM-Extrakt 1) entnommen werden.

Das Raffinat, das die an 5-HMF verarmte wässrige Lösung bildet und welches den Hauptanteil an Nebenprodukten mit einer hohen Wasserlöslichkeit wie Lävulinsäure und andere Carbonsäuren sowie Zucker und Zuckerderivate enthält, wird über die Ableitung (6) aus der Extraktionskolonne (2) entfernt. Aus dem Raffinat können schließlich verschiedene Nebenprodukte gewonnen werden.

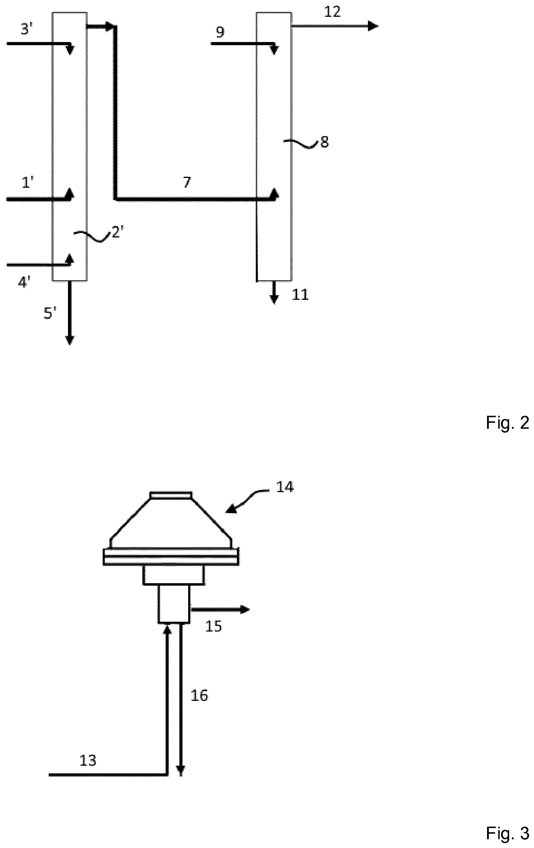

Fig. 2 zeigt schematisch den Ablauf einer Extraktion von 5-HMF mittels inverser Extraktion und Flüssig-flüssig-Extraktion mit zwei hintereinander geschalteten Extraktionskolonnen aus der wässrigen Lösung, die zu der wässrigen Lösung aus Fig.1 identisch ist (Tabelle 1, HMF-Lösung). Die wässrige Lösung wird nach einer Filtration zunächst über die Zuleitung (1') im Bodenbereich einer Extraktionskolonne (2') eingespeist, die invers (mit umgekehrter Dispergierrichtung) zur nachfolgenden betrieben wird. Das Extraktionsmittel Dichlormethan wird über die Zuleitung (3') am Kopf der Extraktionskolonne (2') eingeführt und bei Raumtemperatur im Gegenstrom zur wässrigen Lösung geleitet.

Ein Rückstrom aus Wasser zur Rückextraktion von 5-HMF aus Dichlormethan wird der Extraktionskolonne (2') über die Zuleitung (4') zugeführt. Die Rückextraktion erfolgt durch Vermischen des beladenen Dichlormethans an einer Stelle stromabwärts - in Bezug auf die Strömungsrichtung des Dichlormethans - der Stelle, an der die wässrige Lösung in die Kolonne eingebracht wird. Das Wasser wird hierzu in die Kolonne an einer Stelle unterhalb des Zufuhrpunktes der wässrigen Lösung eingebracht und bildet einen zum Dichlormethan gegenläufigen Rückstrom.

Der inversen Extraktionskolonne (2') wird über die Ableitung (5') mit FHK, Furfural und löslichen Furan-Oligomeren angereichertes Dichlormethan als ein erster Extrakt entnommen.

Ein erstes Raffinat, welches 5-HMF enthält, gelangt über die von dem Kopf der inversen Extraktionskolonne (2') ausgehende Leitung (7) in eine weitere Flüssig-flüssig-Extraktionskolonne (8), bei der es im Bodenbereich eingespeist wird. Die Flüssig-flüssig-Extraktionskolonne (8) wird mit derselben Dispergierrichtung geführt, wie die Extraktionskolonne (2) der Fig. 1. Dichlormethan wird über die Zuleitung (9) am Kopf der zweiten Extraktionskolonne (8) eingeführt und bei Raumtemperatur im Gegenstrom zum ersten Raffinat geleitet.

Über eine Ableitung (11) am Boden der Extraktionskolonne (8) wird ein zweiter Extrakt entnommen, der ca. 7,5 Gew.% bis 10 Gew.% 5-HMF mit einer Reinheit von 97% bis 99% in Dichlormethan enthält. Die Anteile der im zweiten Extrakt enthaltenen Komponenten im Verhältnis zu 5-HMF können der Tabelle 1 (DCM-Extrakt 2) entnommen werden.

Das zweite Raffinat, welches den Hauptanteil an Nebenprodukten mit einer hohen Wasserlöslichkeit wie Lävulinsäure und andere Carbonsäuren sowie Zucker und Zuckerderivate enthält, wird über die Ableitung (12) aus der Extraktionskolonne (8) entfernt.

Fig. 3 zeigt schematisch die Aufkonzentrierung von mit 5-HMF beladenem Dichlormethan nach einer Abtrennung von der wässrigen Lösung. Das mit 5-HMF beladene Dichlormethan, das aus den Extraktionskolonnen (2) und (8), die in den Figuren 1 und 2 dargestellt sind, gewonnen wird, gelangt über die Leitung (13) in einen Dünnschicht-Verdampfer (14). Hierbei wird das aus einer Extraktionskolonne (2) oder (8) entnommene, mit 5-HMF beladene Extraktionsmittel jeweils separat in den Dünnschicht-Verdampfer (14) eingeleitet, in welchem ein Teil des Dichlormethans verdampft, kondensiert und anschließend über die Ableitung (15) entfernt wird. Das Dichlormethan kann schließlich erneut den Extraktionskolonnen (2), (2') und/oder (8) zugeführt werden. Nach Eindampfung des aus der Extraktionskolonne (2) erhaltenen 5-HMF in Dichlormethan wird eine 5-HMF-Dichlormethan-Lösung mit ca. 15 Gew.% bis 20 Gew.% 5-HMF und einer Reinheit von 88% bis 90% erhalten. Nach Eindampfen des aus der Extraktionskolonne (8) erhaltenen 5-HMF in Dichlormethan wird eine 5-HMF-Dichlormethan-Lösung mit ca. 15 Gew.% bis 20 Gew.% 5-HMF und einer Reinheit von 97% bis 99% erhalten. Die aufkonzentrierten 5-HMF-Dichlormethan-Lösungen werden über die Ableitung (16) dem Dünnschicht-Verdampfer (14) entnommen.

**Tabelle 1**

| | **HMF-Lösung** | **DCM-Extrakt 1** | **DCM-Extrakt 2** |
|---|---|---|---|
| **Komponenten** | Soweit nicht anders bezeichnet: alle Angaben in Gew.% relativ zu 5-HMF | | |
| 5-HMF | (100) | (100) | (100) |
| Fructose | ca. 1 | <0,1 | <0,1 |
| Glucose und Zuckerintermediate | ca. 5 | <0,1 | <0,1 |
| Furyl-Hydroxymethyl-Keton (FHK) | 5 | ca. 5 | 0,5 - 1 |
| Furfural | 4 | ca. 4 | <0,1 |
| | | | |
| Lävulinsäure | 1 - 2 | <0,1 | <0,1 |
| Essigsäure | 0,5 - 1 | <0,1 | <0,1 |
| Ameisensäure | 0,5 - 1 | <0,1 | <0,1 |
| | | | |
| Formaldehyd | ca. 1 | <0,1 | <0,1 |
| Ionen (Nitrat, Natrium, etc.) | 0,25-0,3 | <10 ppm | <10 ppm |
| nicht näher untersuchte organische Nebenprodukte mit niedrigem Molekulargewicht | ca. 1 | <1 | <0,1 |
| lösliche Oligomere | ca. 2 | 1 - 2 | <0,1 |

**Tabelle 2**

| | **DCM-Extrakt 1** | **DCM-Extrakt 2** |
|---|---|---|
| Reinheit HMF [%] | 88 bis 90 | 97 bis 99 |

In der Tabelle 2 ist die hohe Reinheit des 5-HMF in den Extrakten 1 und 2 dargestellt. 5-HMF dieser Reinheit eignet sich hervorragend als Plattformchemikalie für eine Reihe industrieller Anwendungen und dient beispielsweise als Vorstufe für die Synthese verschiedener nicht-mineralölbasierter Lösungsmittel, Tenside, Pharmazeutika, Pflanzenschutzmittel und Verbindungen zur Herstellung von Polymeren. 2,5-Furandicarbonsäure und 2,5-Diformylfuran sind derartige Verbindungen, die beispielsweise durch Oxidation aus dem 5-HMF erhalten werden können.

Weitere Vorteile und vorteilhafte Ausgestaltungen sind den Ansprüchen zu entnehmen. Sämtliche Merkmale der Erfindung können sowohl einzeln als auch in beliebiger Kombination miteinander erfindungswesentlich sein.

### BEZUGSZEICHENLISTE

- 1,1': Zuleitung
- 2: Extraktionskolonne
- 2': inverse Extraktionskolonne
- 3, 3': Zuleitung
- 4': Wasch-Rückstrom
- 5, 5': Ableitung
- 6: Ableitung
- 7: Leitung
- 8: Extraktionskolonne
- 9: Zuleitung
- 10 11: Ableitung
- 12: Ableitung
- 13: Leitung
- 14: Dünnschicht-Verdampfer
- 15: Ableitung
- 16: Ableitung

## Patentansprüche

1. Verfahren zur Gewinnung von 5-HMF aus einer wässrigen Lösung umfassend die Schritte
(A) Bereitstellen der wässrigen Lösung enthaltend 5-HMF, und eines organischen Extraktionsmittels,
(B) Flüssig-flüssig-Extraktion, bei der die wässrige Lösung mit dem organischen Extraktionsmittel unter Ausbildung einer Dispersion durchmischt wird, wobei das organische Extraktionsmittel so bemessen ist, dass sich beim Dispergieren eine wässrige Raffinatphase b1 als disperse Phase und eine organische Phase b2 als kontinuierliche Phase ausbildet, wobei eine im Wesentlichen von 5-HMF freie wässrige Raffinatphase b1 und eine mit 5-HMF beladene organische Phase b2 erhalten werden,
(C) Abtrennen der organischen Phase b2 von der wässrigen Raffinatphase b1.

2. Verfahren nach Anspruch 1, **dadurch gekennzeichnet, dass** die wässrige Lösung Fructose, Glucose, Zuckerintermediate und Carbonsäuren enthält und dass in Schritt (B) eine wässrige Raffinatphase b1 enthaltend Fructose, Glucose, Zuckerintermediate und Carbonsäuren erhalten wird.

3. Verfahren nach Anspruch 1 oder 2, **dadurch gekennzeichnet, dass** das organische Extraktionsmittel ausgewählt ist aus
- den halogenierten Kohlenwasserstoffen und insbesondere dem Dichlormethan und dem Chloroform,
- den Ketonen und insbesondere dem Methylisobutylketon,
- den aliphatischen Kohlenwasserstoffen und insbesondere dem 1-Butanol und dem 2-Butanol,
- den Furan-Derivaten und insbesondere dem 2-Methyltetrahydrofuran und dem Tetrahydrofuran,
- den alkylierten Phenolen, insbesondere dem o-Propylphenol und dem o-Isopropylphenol,
oder aus Mischungen von diesen.

4. Verfahren nach einem der Ansprüche 1 bis 3, **dadurch gekennzeichnet, dass** das organische Extraktionsmittel ausgewählt ist aus Dichlormethan und Mischungen enthaltend Dichlormethan.

5. Verfahren nach einem der vorhergehenden Ansprüche, **dadurch gekennzeichnet, dass** vor der Durchführung von Schritt (B) eine Flüssig-flüssig-Extraktion mit umgekehrter Dispergierrichtung (inverse Extraktion) durchgeführt wird, bei der die wässrige Lösung mit dem organischen Extraktionsmittel unter Ausbildung einer Dispersion durchmischt wird, wobei das organische Extraktionsmittel so bemessen ist, dass sich beim Dispergieren eine wässrige Raffinatphase i1 als kontinuierliche Phase und eine organische Phase i2 als disperse Phase ausbildet.

6. Verfahren nach Anspruch 5, **dadurch gekennzeichnet, dass** die wässrige Lösung 5-HMF, Furyl-Hydroxymethyl-Keton (FHK) und Furfural, sowie lösliche Furan-Oligomere enthält und eine wässrige Raffinatphase i1 enthaltend 5-HMF und eine mit FHK, Furfural sowie löslichen Furan-Oligomeren beladene organische Phase i2 erhalten wird.

7. Verfahren nach Anspruch 6, **dadurch gekennzeichnet, dass** bei der Durchführung der inversen Extraktion eine Rückextraktion mit einem polaren Lösungsmittel durchgeführt wird, um in die organische Phase i2 übergetretenes 5-HMF vollständig oder partiell aus der organischen Phase i2 in die wässrige Raffinatphase i1 rückzuführen.

8. Verfahren nach Anspruch 7, **dadurch gekennzeichnet, dass** das 5-HMF rückextrahiert wird, indem die organische Phase i2 mit dem polaren Lösungsmittel in innigen Kontakt gebracht wird, wobei 5-HMF aus der organischen Phase i2 in das polare Lösungsmittel überführt wird, und das mit 5-HMF beladene polare Lösungsmittel mit der wässrigen Raffinatphase i1 vereint wird.

9. Verfahren nach Anspruch 7 oder 8, **dadurch gekennzeichnet, dass** das polare Lösungsmittel Wasser ist.

10. Verfahren nach einem der vorhergehenden Ansprüche, **dadurch gekennzeichnet, dass** eine oder mehrere Extraktionen im Gegenstrom vorgenommen werden.

11. Verfahren nach einem der vorhergehenden Ansprüche, **dadurch gekennzeichnet, dass** das erfindungsgemäße Verfahren den folgenden weiteren Schritt umfasst
(D) partielles oder vollständiges Entfernen des in der organischen Phase b2 enthaltenen organischen Extraktionsmittels.

12. Verfahren nach einem der vorhergehenden Ansprüche, **dadurch gekennzeichnet, dass** eingangs des Verfahrens eine Filtration der wässrigen Lösung vorgenommen wird.
